# EUROPEAN PATENT APPLICATION

(11) **EP 4 799 574 A1**
(43) Date of publication of application: **02.09.2026**
(21) Application number: 25223048.7
(22) Date of filing: 12.12.2025
(51) Int. Cl.: A61B 7/04

(54) **ELECTRONIC DEVICE**

(30) Priority: 26.02.2025 JP 2025028785; 02.06.2025 JP 2025091535
(71) Applicant: Onkyo Corporation, Osaka-shi Osaka 542-0081 (JP)
(72) Inventor: TAKESHIMA, Yoshitada, Osaka-shi, Osaka, 542-0081 (JP); KITAGAWA, Norimasa, Osaka-shi, Osaka, 542-0081 (JP); FUJITANI, Takeshi, Osaka-shi, Osaka, 542-0081 (JP)
(74) Representative: HKW Intellectual Property PartG mbB

(57) **Abstract**

A stethoscope 1 includes a piezoelectric element 4 and a holder 3 which sandwiches and holds the piezoelectric element 4 from an outer circumference of the piezoelectric element 4. The holder 3 has a base 3a and a holding part 3b which bends from an outer circumference of the base 3a to an inside of the base 3a. In a state that the outer circumference of the piezoelectric element 4 is fitted into a space between the base 3a and the holding part 3b, the holder 3 sandwiches and holds the outer circumference of the piezoelectric element 4. An enclosure 2 has an opening which corresponds to an outer shape of the base 3a of the holder 3 and an engaging claw 3c of the holder 3 is engaged with an edge of the opening. Thus, a means for mounting an element by simple structure can be provided.

## Description

### TECHNICAL FIELD

The present invention relates to an electronic device.

### BACKGROUND ART

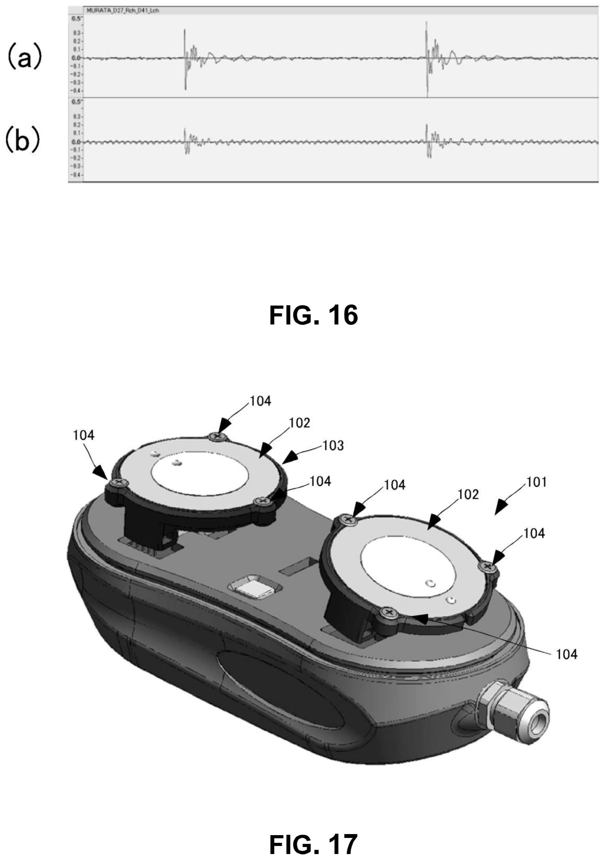

Among stethoscopes, there is a stethoscope which is called an electronic stethoscope which electronically collects a sound such as a heart sound by a sensor such as a microphone, amplifies the collected sound and makes a doctor listen to the amplified sound (for example, see patent literature 1). In a stethoscope described in patent literature 1, for collecting sound, a piezoelectric element is used. Fig. 17 is a perspective diagram illustrating a conventional stethoscope. In Fig. 17, a cover which covers a piezoelectric element 102 and the like is removed from the stethoscope 101. As illustrated, the piezoelectric element 102 is mechanically fixed to the holder 103 by the screw 104.

### PRIOR ART DOCUMENT

### PATENT LITERATURE

PATENT LITERATURE 1: JP 2024-027219A

### SUMMARY OF THE INVENTION

### PROBLEM TO BE RESOLVED BY THE INVENTION

In the conventional stethoscope, since the piezoelectric element is fixed to the holder by the screw, space for the screw is necessary. Further, since there is unevenness in a flat surface by the screw and resulting in the occurrence of unevenness in a cover which covers the piezoelectric element as well, it also affects sound collection. Further, at the time of manufacturing, controlling the strength of screwing of the screws is necessary and assembly management is difficult. In other words, conventional stethoscope has a complex structure that uses screws to attach piezoelectric elements, leading to various problems.

An objective of the present invention is to provide a means for mounting an element by simple structure.

### MEANS FOR SOLVING THE PROBLEM

An electronic device of a first invention comprising: an element; and a holder which sandwiches and holds the element from an outer circumference of the element.

In the present invention, the element is sandwiched and held by the holder. Thus, the element can be mounted on the electronic device by simple structure without using a screw or the like.

The electronic device of a second invention is the electronic device of the first invention, wherein the holder has a base and a holding part which bends from an outer circumference of the base toward an inside of the base.

The electronic device of a third invention is the electronic device of the second invention, wherein the holder sandwiches and holds the outer circumference of the element in a state in which the outer circumference of the element is fitted in a space between the base and the holding part.

The electronic device of a fourth invention is the electronic device of the second invention, wherein the base of the holder is a contact surface for contact with a subject from which sound is collected in a state of collecting the sound by the element.

The electronic device of a fifth invention comprising: an element; and a holder which holds the element, wherein the holder has an engaging claw which is engaged with an enclosure.

In the present invention, the holder which holds the element has the engaging claw which is engaged with the enclosure. Thus, since the holder which holds the element is mounted on the enclosure of the electronic device by the engaging claw, the element can be mounted on the electronic device by simple structure without using a screw or the like.

The electronic device of a sixth invention is the electronic device of the fifth invention, wherein the holder has a base and the engaging claw which projects to an outside of the base.

The electronic device of a seventh invention is the electronic device of the sixth invention, wherein the enclosure has an opening which corresponds to an outer shape of the base of the holder, and the engaging claw of the holder is engaged with an edge of the opening.

An electronic device of an eighth invention comprising: an element; a holder which holds the element; and an enclosure on which the holder is mounted, wherein the holder has a base, a holding part which bends from an outer circumference of the base toward an inside of the base and an engaging claw which projects from the outer circumference of the base to an outside of the base.

In the present invention, the holder which holds the element has the holding part which bends from the outer circumference of the base toward the inside of the base. For this reason, the element is fitted into a space between the holding part and the base and is held by the holder. Further, the holder has the engaging claw which projects from the outer circumference of the base toward the outside of the base. For this reason, the holder which holds the element is mounted on the enclosure by the engaging claw. Thus, in the present invention, the element can be mounted on the electronic device by simple structure without using a screw or the like.

The electronic device of a ninth invention comprising: an element; a holder which holds the element; and an enclosure on which the holder is mounted, wherein the holder exposes from an opening of the enclosure, the enclosure has tapers, and one taper extends from the opening to one direction in a longitudinal direction of the enclosure and the other taper extends from the opening to the other direction in the longitudinal direction of the enclosure.

In a state in which the element collects sound, the holder which exposes from the opening of the enclosure becomes a contact surface for contact with a subject from which sound is collected. In the present invention, the enclosure has the tapers. One taper extends from the opening to one direction in the longitudinal direction of the enclosure and the other taper extends from the opening to the other direction in the longitudinal direction of the enclosure. For this reason, even if the subject from which sound is collected has unevenness, it is easy to contact the contact surface for contact with the subject when using the electronic device.

The electronic device of a tenth invention is the electronic device of the first, the fifth, the eighth or the ninth invention, wherein the holder has elasticity.

The electronic device of an eleventh invention is the electronic device of the first, the fifth, the eighth or the ninth invention, wherein the element is a piezoelectric element.

The electronic device of a twelfth invention is the electronic device of the first, the fifth, the eighth or the ninth invention, wherein the element has a substantially circular shape with a predetermined thickness.

The electronic device of a thirteenth invention is the electronic device of the first, the fifth, the eighth or the ninth invention, wherein the electronic device is a stethoscope.

The electronic device of a fourteenth invention is the electronic device of the first invention, wherein the electronic device further comprising: an enclosure which has an opening corresponding to the holder; a stopper; and a screwing member, wherein in a state in which an outer circumference of the holder abuts on an edge of the opening and the stopper and the edge of the opening sandwich the holder, the screwing member is screwed to the enclosure via the stopper.

In the present invention, the enclosure has the opening corresponding to the holder. In the state in which the outer circumference of the holder abuts on the edge of the opening and the stopper and the edge of the opening sandwich the holder, the screwing member is screwed to the enclosure via the stopper. Thus, in a state in which the holder is mounted on the enclosure, a gap between the holder and the enclosure can be eliminated.

### EFFECT OF THE INVENTION

According to the present invention, an element can be mounted on an electronic device by simple structure.

### BRIEF DESCRIPTION OF THE DRAWINGS

Fig. 1 is a perspective diagram illustrating a stethoscope according to an embodiment of the present invention.
Fig. 2 is a perspective diagram illustrating the stethoscope according to the embodiment of the present invention.
Fig. 3 is a front diagram illustrating the stethoscope according to the embodiment of the present invention.
Fig. 4 is a rear diagram illustrating the stethoscope according to the embodiment of the present invention.
Fig. 5 is a top diagram illustrating the stethoscope according to the embodiment of the present invention.
Fig. 6 is a bottom diagram illustrating the stethoscope according to the embodiment of the present invention.
Fig. 7 is a right side diagram illustrating the stethoscope according to the embodiment of the present invention.
Fig. 8 is a left side diagram illustrating the stethoscope according to the embodiment of the present invention.
Fig. 9 is a cross-sectional diagram in a direction which is orthogonal to a longitudinal direction of the stethoscope.
Fig. 10 is an enlarged diagram of a main part in Fig. 9.
Fig. 11 is a perspective diagram illustrating a ground plate.
Fig. 12 is a cross-sectional diagram in the direction which is orthogonal to the longitudinal direction of the stethoscope.
Fig. 13 is an enlarged diagram of a main part in Fig. 12.
Fig. 14 is a perspective diagram illustrating the stethoscope in a state in which an inside of the stethoscope can be seen.
Fig. 15 is a scheme diagram illustrating a state in which a piezoelectric element and a substrate are connected by a double-core shielded wire.
Fig. 16 is a diagram for describing difference of sensitivity by size difference of the piezoelectric element.
Fig. 17 is a perspective diagram illustrating a conventional stethoscope.
Fig. 18 is a perspective diagram illustrating a conventional stethoscope.
Fig. 19 is a cross-sectional diagram in a direction which is orthogonal to a longitudinal direction of a stethoscope according to a variation.
Fig. 20 is a perspective diagram illustrating a holder.
Fig. 21 is a perspective diagram illustrating a stopper.

### DESCRIPTION OF THE EMBODIMENTS

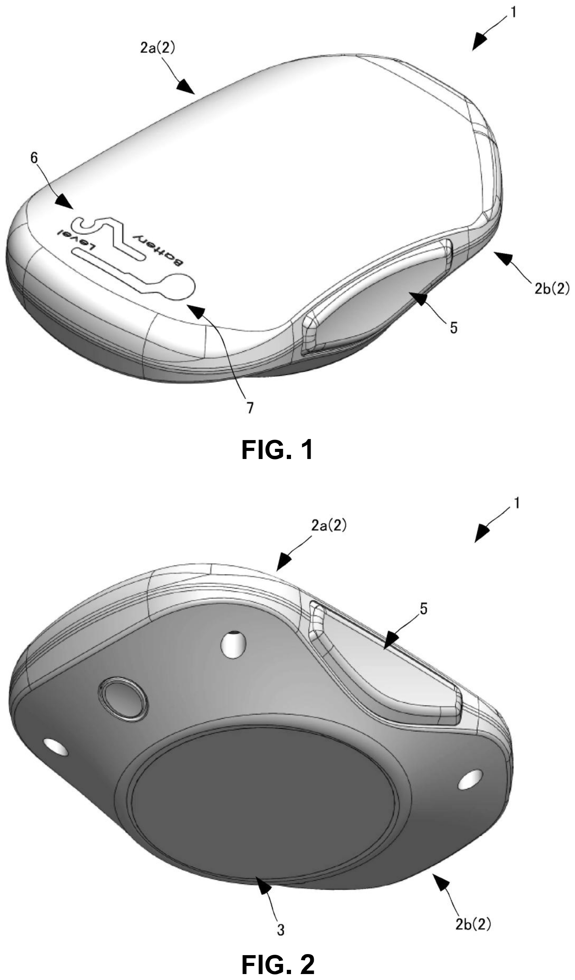

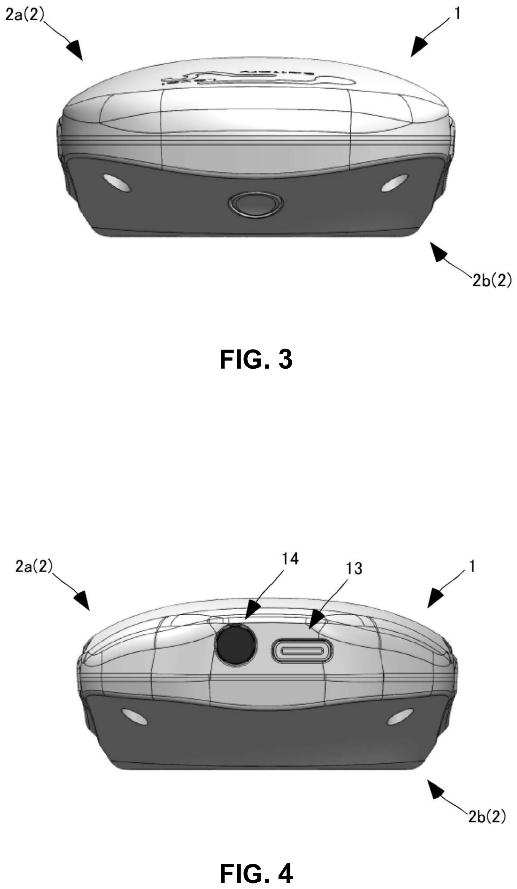

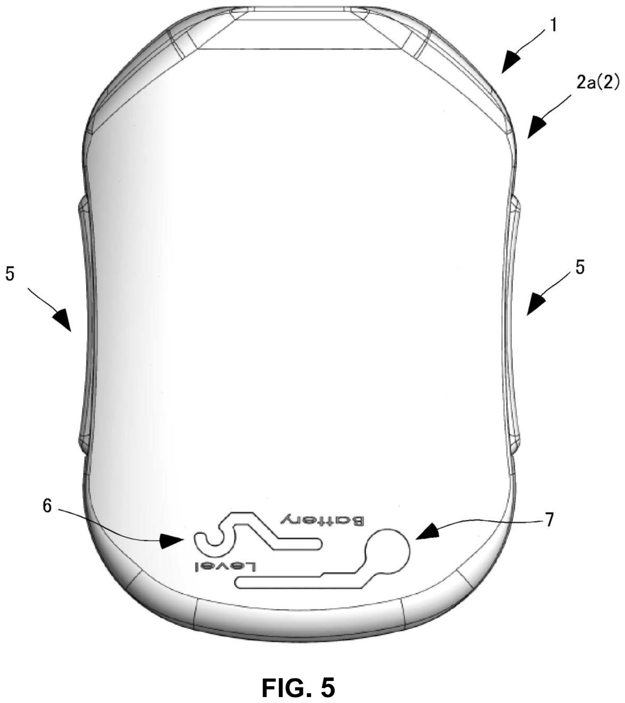

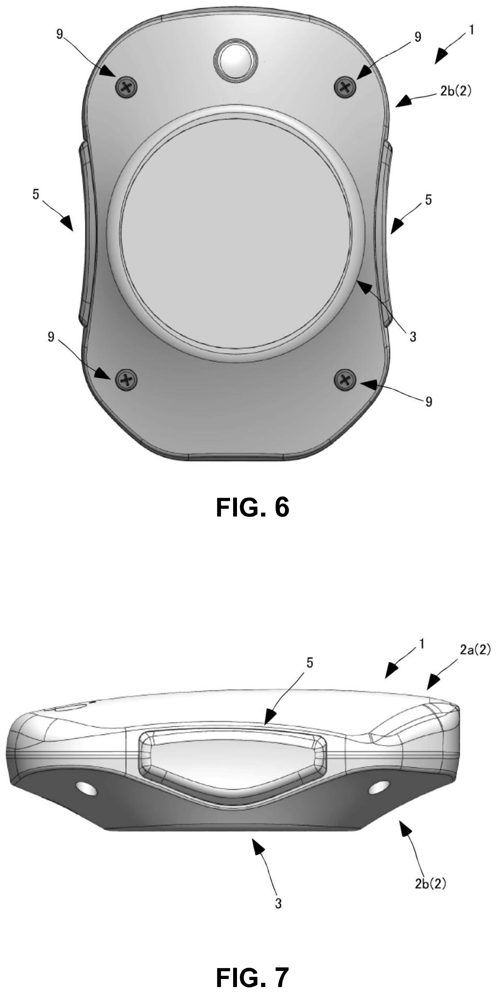

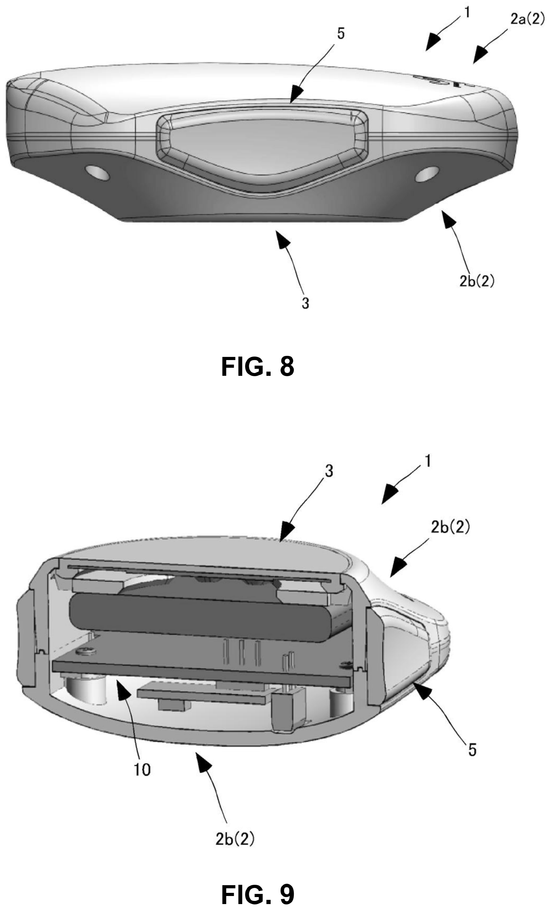

An embodiment of the present invention is described below. Each of Fig. 1 and Fig. 2 is a perspective diagram illustrating a stethoscope 1 (an electronic device) according to an embodiment of the present invention. Fig. 3 is a front diagram illustrating the stethoscope 1 according to the embodiment of the present invention. Fig. 4 is a rear diagram illustrating the stethoscope 1 according to the embodiment of the present invention. Fig. 5 is a top diagram illustrating the stethoscope 1 according to the embodiment of the present invention. Fig. 6 is a bottom diagram illustrating the stethoscope 1 according to the embodiment of the present invention. Fig. 7 is a right side diagram illustrating the stethoscope 1 according to the embodiment of the present invention. Fig. 8 is a left side diagram illustrating the stethoscope 1 according to the embodiment of the present invention.

The stethoscope 1 collects a heart sound, a lung sound and the like for auscultation. A user of the stethoscope 1 uses the stethoscope 1 by grasping the stethoscope 1 from a top surface side of the stethoscope 1 by his or her hand and contacting a bottom surface side of the stethoscope 1 to an auscultatory subject. A piezoelectric element 4 is provided inside and at the bottom surface side of the stethoscope 1.

The stethoscope 1 includes an enclosure 2, a holder 3, the piezoelectric element 4, a ground plate 5 and the like. The enclosure 2 (the stethoscope 1) has a substantially rectangular shape and four corner parts of the substantially rectangular shape are chamfered. Each of both sides which are orthogonal to a longitudinal direction of the enclosure 2 is recessed to an inside direction. A substrate 10 and the like are stored inside the enclosure 2. The enclosure 2 consists of an upper enclosure 2a and a lower enclosure 2b. The upper enclosure 2a and the lower enclosure 2b are screwed by four screws 9. The screw 9 is inserted from the bottom surface side of the lower enclosure 2b into the upper enclosure 2a side and screws the upper enclosure 2a and the lower enclosure 2b. In the lower enclosure 2b, at the bottom surface side, an opening for which the holder 3 is provided is provided. The holder 3 exposes from the opening of the enclosure 2. The enclosure 2 has tapers. One taper is from the opening toward one direction (a front direction) in a longitudinal direction of the enclosure 2 and the other taper is from the opening toward the other direction (a rear direction) in the longitudinal direction of the enclosure 2. Namely, the enclosure 2 becomes a taper shape from the opening toward the front direction and a taper shape from the opening toward the rear direction.

At the top surface side of the stethoscope 1, a battery meter 6 which indicates the remaining capacity of a battery and a level meter 7 which indicates the level of collected sound are provided. Further, at the rear surface side of the stethoscope 1, a USB port 13 and an earphone jack 14 are provided.

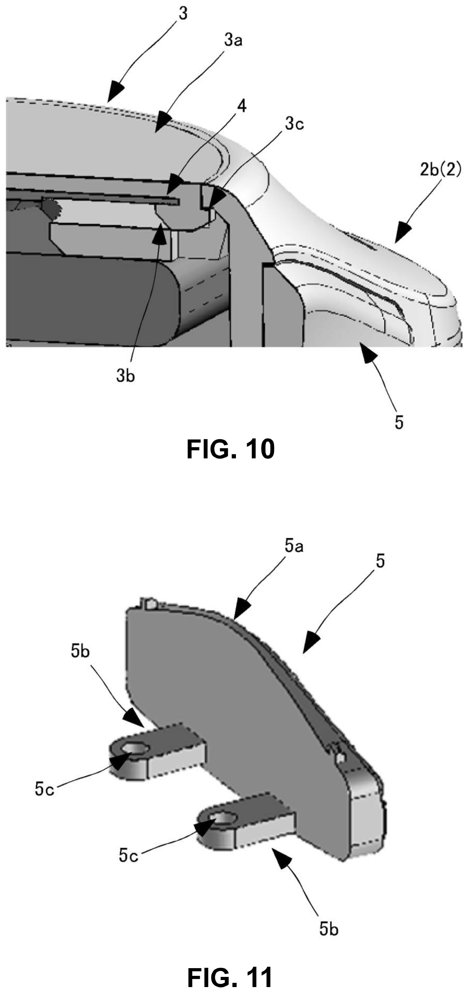

Fig. 9 is a cross-sectional diagram in a direction which is orthogonal to the longitudinal direction of the stethoscope 1. Fig. 10 is an enlarged diagram of a main part in Fig. 9. The holder 3 is for holding the piezoelectric element 4. For example, the holder 3 is made of a rubber which has elasticity. The holder 3 is circular when seen from top. The holder 3 has a base 3a, a holding part 3b and an engaging claw 3c. The base 3a is a circular shape part with a predetermined thickness. The holding part 3b is a part which holds the piezoelectric element 4. The holding part 3b bends from an outer circumference toward an inside. There is a space between the base 3a and the holding part 3b. The piezoelectric element 4 is fitted into the holding part 3b and the outer circumference of the piezoelectric element 4 is held by the holding part 3b. Namely, in a state in which the outer circumference of the piezoelectric element 4 is fitted into the space between the base 3a and the holding part 3b, the holder 3 sandwiches and holds the outer circumference of the piezoelectric element 4. The engaging claw 3c is for engaging the holder 3 with the lower enclosure 2b (the enclosure 2). The engaging claw 3c is engaged with the edge of the opening of the enclosure 2. The holder 3 is mounted on the lower enclosure 2b by the engaging claw 3c. For example, the six engaging claws 3c are provided at equal intervals in a circumferential direction at the holder 3 which is circular when seen from top.

The piezoelectric element 4 is flat and substantially disk-shaped. The piezoelectric element 4 has a predetermined thickness. The piezoelectric element 4 is mounted on the holder 3. In the embodiment, the holder 3 becomes a contact surface which contacts a human body (a subject). The piezoelectric element 4 converts sound (vibration) from the human body into voltage via the holder 3.

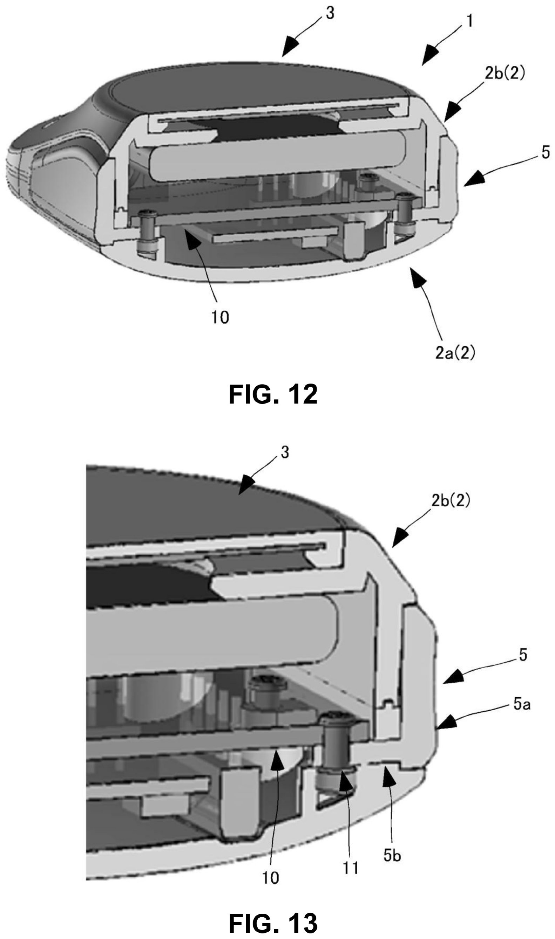

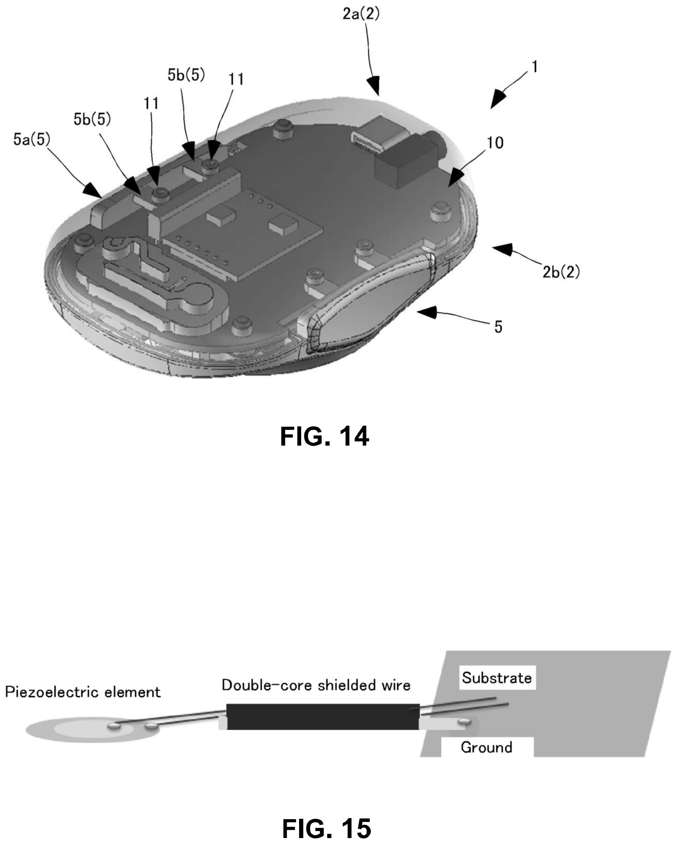

Fig. 11 is a perspective diagram illustrating the ground plate 5. Fig. 12 is a cross-sectional diagram in the direction which is orthogonal to the longitudinal direction of the stethoscope 1. Fig. 13 is an enlarged diagram of a main part in Fig. 12. Fig. 14 is a perspective diagram illustrating the stethoscope 1 in a state in which an inside of the stethoscope can be seen. Two ground plates 5 are provided at both sides of a direction which is orthogonal to the longitudinal direction of the enclosure 2 (the stethoscope 1). The ground plate 5 is provided at recessed parts of both sides which are orthogonal to the longitudinal direction of the enclosure 2. The ground plate 5 exposes from the enclosure 2 externally. The ground plate 5 has a substantially pentagonal shape when seen from top. The ground plate 5 has a base 5a and two arms 5b. The arm 5b projects from an extending direction of the base 5a to a vertical direction of the base 5a. A screw hole 5c is provided at a tip of the arm 5b. The arm 5b extends substantially parallel to a direction to which the substrate 10 extends. Namely, the arm 5b and the substrate 10 are substantially parallel each other. The ground plate 5 is fixed to the substrate 10 by a screw 11 which is inserted into the screw hole 5c. Namely, the arm 5b and the substrate 10 which are substantially parallel each other are screwed by the screw 11 (a screwing member) which extends to a direction perpendicular to the arm 5b and the substrate 10.

Further, the piezoelectric element 4 is connected to ground on the substrate 10 by a double-core shielded wire as illustrated in Fig. 15 schematically.

The circuitry including an amplifier which is for amplifying sound collected by the piezoelectric element 4 is mounted on the substrate 10.

Fig. 16 is a diagram for describing difference of sensitivity by size difference of the piezoelectric element. Fig. 16(a) illustrates level of a piezoelectric element having a diameter of 41 mm. Fig. 16(b) illustrates level of a piezoelectric element having a diameter of 27 mm. Herein, a weight is mounted on a piezoelectric element put on ground and vibration of ground is measured by the piezoelectric element. As illustrated, level of the piezoelectric element having the diameter of 41 mm is larger than level of the piezoelectric element having the diameter of 27 mm. From this, it is understood that sensitivity of a piezoelectric element having a larger diameter is higher as compared to a smaller one.

Conventionally, as illustrated in Fig. 17, since a piezoelectric element 102 is fixed to a holder 103 by a screw 104 mechanically, a space for the screw 104 is necessary at an outer circumference of the piezoelectric element 102. Further, since the screw 104 is used, there is unevenness around the piezoelectric element 102 and on a cover which covers the piezoelectric element 102. Further, in case of fixing by the screw 104, controlling of the strength of screwing the screw 104 is necessary and management of assembly is difficult.

As described above, the holder 4 has the holding part 4b which bends from the outer circumference of the base 4a to the inside of the base 4a. By the holding part 4b, the outer circumference of the piezoelectric element 3 is sandwiched and held, a bottom surface of the piezoelectric element 3 is covered by the holder 4 and falling from the stethoscope 1 is prevented. In the embodiment, since a screw is not used for mounting the piezoelectric element 3 on the holder 4 and space for screwing is not necessary, space is saved. Thus, a piezoelectric element in which sensitivity is high and a diameter is large can be used. Further, since there is no unevenness around the piezoelectric element 3 and on a surface of the holder 4 which covers the piezoelectric element 3, sound can be collected well when the surface of the holder 4 of the stethoscope 1 is contacted to the human body which is an auscultatory subject. Further, since a screw is not used, controlling of the strength of screwing the screw is not necessary and assemblability is improved.

The holder 4 has the engaging claw 4c which projects from the outer circumference of the base 4a. The engaging claw 4c is engaged with the edge of the opening of the enclosure 2 (the lower enclosure 2b) and the piezoelectric element 3 which is held by the holder 4 is mounted on the enclosure 2 (the lower enclosure 2b). Further, for collecting sound, it is important that the piezoelectric element 3 (the holder 4) contacts the human body which is the auscultatory subject. By saving space, it is easy that the holder 4 (the piezoelectric element 3) follows unevenness of the human body which is the auscultatory subject and fits to the human body. In addition, since a screw is not used for mounting the holder 4 on the enclosure 2, the number of parts is increased and assemblability of the stethoscope 1 is improved.

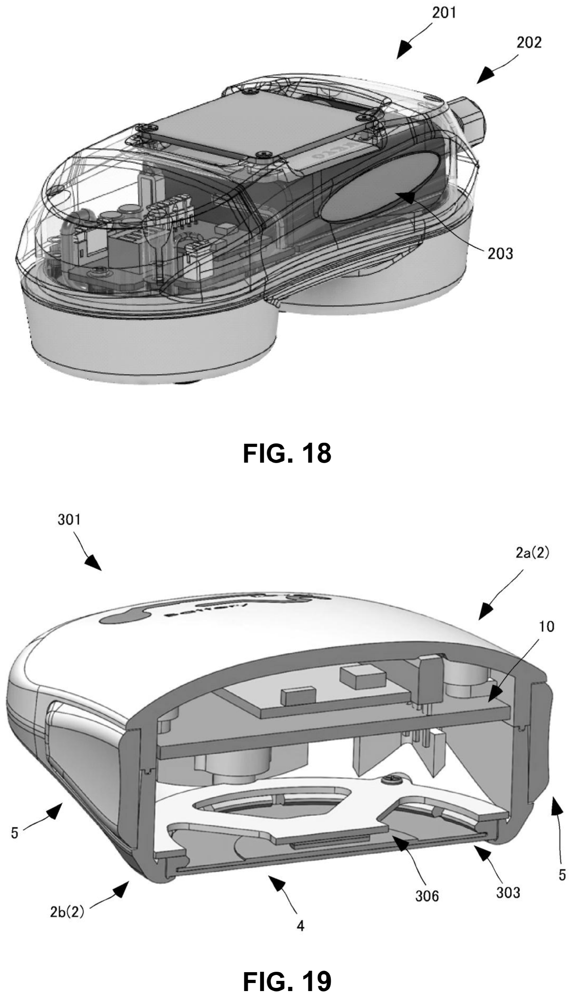

Conventionally, as illustrated in Fig. 18, in a stethoscope 201, a ground plate 203 is provided so that the ground plate 203 exposes from an enclosure 202. Wiring against the ground plate is a structure that a wiring is soldered to an inside of a sheet metal member and the wiring is connected to an internal substrate. According to this structure, the wiring may be disconnected or bitten and assemblability may be spoiled. Further, since the sheet metal is used, there is limitation for design.

In the embodiment, since the ground plate 5 contacts the substrate 10 by the arm 5b directly and is securely fixed to the substrate 10 by the screw 11, thereby ensuring reliable grounding. Further, since the ground plate 5 is provided at each of both sides which are orthogonal to the longitudinal direction of the enclosure 2 and the part which is recessed to the inside, the user's hand contacts the ground plate 5 naturally when using the stethoscope 1 and ground can be performed certainly. Further, it is easy for the user to grasp the recessed part.

Further, conventionally, since wiring of a piezoelectric element becomes a loop shape like an antenna and is easy to pick noise up, it is necessary to care about a wiring route. Further, in wiring, since impedance is high, there is a case where noise superimposes by electrical combination when the wiring is naked.

In the embodiment, the piezoelectric element 4 is connected to ground on the substrate 10 by the double-core shielded wire. Thus, mixing of external noise is prevented.

As described above, in the embodiment, the piezoelectric element 4 is sandwiched and held by the holder 3. Thus, without using a screw or the like, the piezoelectric element 4 can be mounted on the stethoscope 1 by simple structure.

Further, the holder 3 which holds the piezoelectric element 4 has the engaging claw 3c which is engaged with the enclosure 2. Thus, since the holder 3 which holds the piezoelectric element 4 is mounted on the enclosure 2 of the stethoscope 1 by the engaging claw 3c, the piezoelectric element 4 can be mounted on the stethoscope 1 by simple structure without using a screw or the like.

Further, in the embodiment, the holder 3 which holds the piezoelectric element 4 has the holding part 3b which bends from the outer circumference to the inside of the base 3a. Thus, the piezoelectric element 4 is fitted into the space between the holding part 3b and the base 3a and held by the holder 3. Further, the holder 3 has the engaging claw 3c which projects from the outer circumference of the base 3a to the outside of the base 3a. Therefore, the holder 3 which holds the piezoelectric element 4 is mounted on the enclosure 2 by the engaging claw 3c. Thus, in the embodiment, the piezoelectric element 4 can be mounted on the stethoscope 1 by simple structure without using a screw or the like.

In a state of collecting sound by the piezoelectric element 4, the holder 3 which exposes from the opening of the enclosure 2 becomes a contact surface for contact with a subject from which sound is collected. In the embodiment, the enclosure 2 has tapers. One taper is toward one direction (the front direction) of the longitudinal direction of the enclosure 2 from the opening, and the other taper is toward the other direction (the rear direction) of the longitudinal direction of the enclosure 2 from the opening. Therefore, even if the subject from which sound is collected has unevenness when using the stethoscope 1, it is easy to contact the contact surface for contact with the subject.

Further, in the embodiment, the substrate 10 and the ground plate 5 are screwed by the screw 11. Unlike the conventional invention, since a substrate and a ground plate are not connected by a wiring, ground can be performed by simple structure and mixing of hum noise can be prevented.

Further, in the embodiment, each of both sides which are orthogonal to the longitudinal direction of the enclosure 2 is recessed to the inside. Therefore, it is easy for the user of the stethoscope 1 to use the stethoscope 1 by grasping the recessed part of a side wall.

Since a piezoelectric element is high impedance, it is easy that the hum noise mixes. In the embodiment, although the stethoscope 1 includes the piezoelectric element 4, mixing of the hum noise can be prevented by the ground plate 5.

Since an amplifier is high impedance, it is easy that the hum noise mixes. In the embodiment, although the stethoscope 1 includes the amplifier, mixing of the hum noise can be prevented by the ground plate 5.

Further, in the embodiment, ground on the substrate and the piezoelectric element are connected by the double-core shielded wire. Thus, mixing of the hum noise can be prevented.

A stethoscope according to a variation is described below. Fig. 19 is a cross-sectional diagram in a direction which is orthogonal to a longitudinal direction of the stethoscope 301 according to the variation. The stethoscope 301 according to the variation includes a holder 303 similarly to the stethoscope 1. However, a mode of the holder 303 is different from the holder 3 of the stethoscope 1. The same signs as the stethoscope 1 are attached to configurations which are similar to the stethoscope 1 and description of similar configurations to the stethoscope 1 in the stethoscope 301 is omitted.

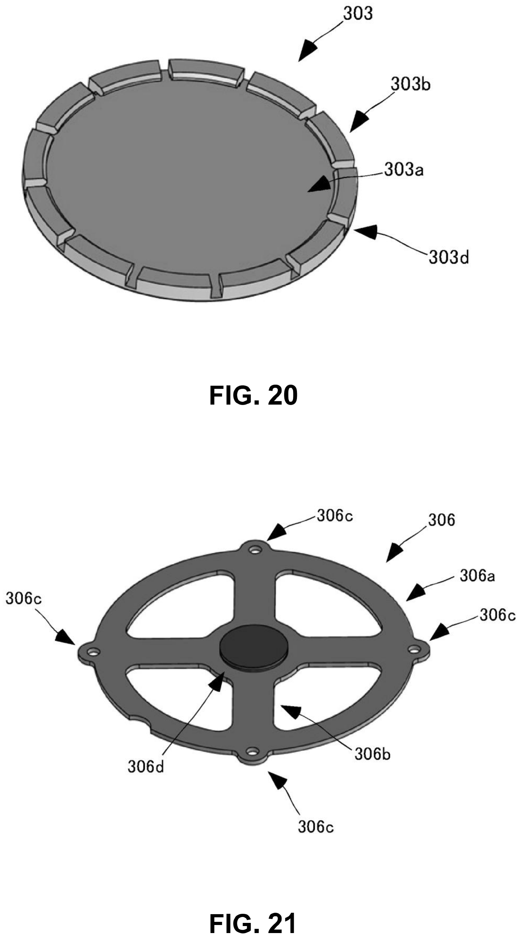

Fig. 20 is a perspective diagram illustrating the holder 303. The holder 303 is for holding the piezoelectric element 4. For example, the holder 303 is made of a rubber which has elasticity. The holder 303 is circular when seen from top. The holder 303 has a base 303a and a holding part 303b. However, the holder 303 is different from the holder 3 and does not have an engaging claw. The base 303a is a circular shape part with a predetermined thickness. The holding part 303b is a part which holds the piezoelectric element 4. The holding part 303b bends from an outer circumference toward an inside. There is a space between the base 303a and the holding part 303b. The piezoelectric element 4 is fitted into the holding part 303b and the outer circumference of the piezoelectric element 4 is held by the holding part 303b. Namely, in a state in which the outer circumference of the piezoelectric element 4 is fitted into the space between the base 303a and the holding part 303b, the holder 303 sandwiches and holds the outer circumference of the piezoelectric element 4. In the holder 303, at the holding part 303b, slits 303d are provided with a predetermined interval. Due to these slits 303d, the piezoelectric element 4 can be inserted into the holder 303. Further, a rubber die is easy to be released.

Although the holder 3 is mounted on the enclosure 2 (the lower enclosure 2b) by the engaging claw 3c in the stethoscope 1, the holder 303 is mounted on the enclosure 2 (the lower enclosure 2b) by a stopper 306 and screws 307 (screwing members) in the stethoscope 301.

Fig. 21 is a perspective diagram illustrating the stopper 306. The stopper 306 has a base 306a which is substantially circular and a base 306b which is substantially +(plus)-shaped. For example, the stopper 306 is made of stainless steel. The stopper 306 may be made of the other material. Four screw holes 306c are provided at the base 306a which is on an extended line of the base 306b with 90 degrees interval. Further, a limitation plate 306d is provided substantially on a center of the base 306b.

When the holder 303 holding the piezoelectric element 4 is mounted on the lower enclosure 2b (the enclosure 2), the holder 303 is put on the lower enclosure 2b so that the outer circumference of holder 303 abuts on the edge of the opening of the lower enclosure 2b first. Next, the stopper 306 is put on the piezoelectric element 4 so that the limitation plate 306d faces to the piezoelectric element 4 and the stopper 306 sandwiches the piezoelectric element 4 with the opening of the lower enclosure 2b. And, the screws 307 are inserted into the screw holes 306c of the stopper 306 from an inside of the enclosure 2 and are screwed to the lower enclosure 2b. Thus, the holder 303 is sandwiched by the stopper 306 and the lower enclosure 2b and mounted on the lower enclosure 2b.

As described above, the limitation plate 306d is provided at the stopper 306. A predetermined gap is provided between the piezoelectric element 4 and the limitation plate 306d. For the purpose of preventing deformation of the piezoelectric element 4 when large burden is on the piezoelectric element 4, the limitation plate 306d is provided. However, the limitation plate 306d may not be provided.

In the variation, the enclosure 2 (the lower enclosure 2b) has the opening corresponding to the holder 303. Further, in a state in which the outer circumference of the holder 303 abuts on the edge of the opening of the lower enclosure 2b and the stopper 306 and the edge of the opening sandwich the holder 303, the screws 307 (the screwing members) are screwed to the enclosure 2 (the lower enclosure 2b) via the stopper 306. Thus, in a state in which the holder 303 is mounted on the enclosure 2, a gap between the holder 303 and the enclosure 2 can be eliminated.

The embodiment of the present invention is described above, but the mode to which the present disclosure is applicable is not limited to the above embodiment and can be suitably varied without departing from the scope of the present disclosure.

### INDUSTRIAL APPLICABILITY

The present invention can be suitably employed in an electronic device.

### DESCRIPTION OF REFERENCE SIGNS

1, 301 stethoscope (electronic device)
2 enclosure
2a upper enclosure
2b lower enclosure
3, 303 holder
3a, 303a base
3b, 303b holding part
3c engaging claw
303d slit
4 piezoelectric element
5 ground plate
5a base
5b arm
5c screw hole
306 stopper
306a, 306b base
306c screw hole
306d limitation plate
307 screw (screwing member)
10 substrate
11 screw (screwing member)

## Claims

1. An electronic device (1, 301) comprising:
an element (4); and
a holder (3, 303) which sandwiches and holds the element (4) from an outer circumference of the element (4).

2. The electronic device (1, 301) according to claim 1,
wherein the holder (3, 303) has a base (3a, 303a) and a holding part (3b, 303b) which bends from an outer circumference of the base (3a, 303a) toward an inside of the base (3a, 303a).

3. The electronic device (1, 301) according to claim 2,
wherein the holder (3, 303) sandwiches and holds the outer circumference of the element (4) in a state in which the outer circumference of the element (4) is fitted in a space between the base (3a, 303a) and the holding part (3b, 303b).

4. The electronic device (1, 301) according to claim 2,
wherein the base (3a, 303a) of the holder (3, 303) is a contact surface for contact with a subject from which sound is collected in a state of collecting the sound by the element (4).

5. An electronic device (1, 301) comprising:
an element (4); and
a holder (3, 303) which holds the element (4),
wherein the holder (3, 303) has an engaging claw (3c) which is engaged with an enclosure (2).

6. The electronic device (1, 301) according to claim 5,
wherein the holder (3, 303) has a base (3a, 303a) and the engaging claw (3c) which projects to an outside of the base (3a, 303a).

7. The electronic device (1, 301) according to claim 6,
wherein the enclosure (2) has an opening which corresponds to an outer shape of the base (3a, 303a) of the holder (3, 303), and
the engaging claw (3c) of the holder (3, 303) is engaged with an edge of the opening.

8. An electronic device (1, 301) comprising:
an element (4);
a holder (3, 303) which holds the element (4); and
an enclosure (2) on which the holder (3, 303) is mounted,
wherein the holder (3, 303) has a base (3a, 303a), a holding part (3b, 303b) which bends from an outer circumference of the base (3a, 303a) toward an inside of the base (3a, 303a) and an engaging claw (3c) which projects from the outer circumference of the base (3a, 303a) to an outside of the base (3a, 303a).

9. The electronic device (1, 301) comprising:
an element (4);
a holder (3, 303) which holds the element (4); and
an enclosure (2) on which the holder (3, 303) is mounted, and
wherein the holder (3, 303) exposes from an opening of the enclosure (2),
the enclosure (2) has tapers, and
one taper extends from the opening to one direction in a longitudinal direction of the enclosure (2) and the other taper extends from the opening to the other direction in the longitudinal direction of the enclosure (2).

10. The electronic device (1, 301) according to claim 1, 5, 8 or 9,
wherein the holder (3, 303) has elasticity.

11. The electronic device (1, 301) according to claim 1, 5, 8 or 9,
wherein the element (4) is a piezoelectric element.

12. The electronic device (1, 301) according to claim 1, 5, 8 or 9,
wherein the element (4) has a substantially circular shape with a predetermined thickness.

13. The electronic device (1, 301) according to claim 1, 5, 8 or 9,
wherein the electronic device (1, 301) is a stethoscope.

14. The electronic device (1, 301) according to claim 1, further comprising:
an enclosure (2) which has an opening corresponding to the holder (3, 303);
a stopper (306); and
a screwing member,
wherein in a state in which an outer circumference of the holder (3, 303) abuts on an edge of the opening and the stopper (306) and the edge of the opening sandwich the holder (3, 303), the screwing member is screwed to the enclosure (2) via the stopper (306).
